# EUROPEAN PATENT SPECIFICATION

(11) **EP 1 637 041 B1**
(45) Date of publication and mention of the grant of the patent: **30.04.2008**
(21) Application number: 04255726.4
(22) Date of filing: 21.09.2004
(51) Int. Cl.: A23K 1/18, A23K 1/16

(54) **Improving longevity of elderly cats**
Verbesserung der Lebensdauer alter Katzen
Amélioration de la longévité de chats âgés

(43) Date of publication of application: 22.03.2006
(73) Proprietor: Nestec S.A., 1800 Vevey (CH)
(72) Inventor: Cupp, Carolyn Jean, Libert MO 64068 (US); Young, Linda A., St. Joseph MO 64506 (US); Czarnecki-Maulden, Easton MO 64443 (US); Perez-Camargo, Cachy 80800 (FR); Patil, Avinash, St. Joseph MO 64507 (US)
(74) Representative: Lock, Graham James

(56) References cited:
- EP-A- 1 457 118
- WO-A-01/17365
- WO-A-01/17366
- WO-A-03/084342
- US-A- 5 952 033
- US-A- 6 156 355
- US-A1- 2001 043 983
- US-A1- 2002 173 450
- US-A1- 2003 035 821
- US-A1- 2003 190 309
- US-A1- 2004 001 875

## Description

### BACKGROUND OF THE INVENTION

This invention relates to a composition for increasing the longevity of elderly cats.

Elderly cats often become frail in their last few years of life. From an appearance point of view, they become thin and have poor skin and coat condition. Other problems/symptoms may include weight changes, changes in hydration status, digestive system problems, decreased blood protein levels, decreased blood hemoglobin levels, decreased red blood cell numbers, joint stiffness and energy loss or lower activity levels in general as compared to younger cats.

Certain of these problems may be effectively treated using medication. However, a better alternative would be to delay the onset of these problems, or treat these problems, through diet adjustments.

WO 01/17366 discloses a method for improving the condition and/or increasing the longevity of elderly pets, wherein a nutritional composition which contains a calcium source and an antioxidant source is administered. US 2001/0043983 discloses pet foods, treats and supplements with anti-ageing effects. The pet foods, treats and supplements include R-α-lipoic acid and L-carnitine. EP 1457118 discloses a composition comprising a source of protein, a source of fat and a source of carbohydrate for controlling post prandial glycemic and/or insulinemic response in companion animals such a dogs. US 2004/0001875 discloses a method for treating abnormal glucose metabolism and insulin resistance in an animal by feeding a diet comprising high protein and moderate amounts of carbohydrate and fat.

WO 01/17365 discloses compositions comprising linoleic acid, chicory and linolenic acid, which are disclosed as being useful for improving the skin and coat condition of pets. Initial findings indicate that animals consuming these compositions also exhibit reduced oxidative stress and inflammation.

However, despite the use of balanced, maintenance foods, the condition of elderly animals may deteriorate rapidly. Therefore there is a need for nutritional ways of increasing the longevity of elderly cats.

### SUMMARY OF THE INVENTION

The present invention provides the use of compositions for increasing the longevity of elderly pets as defined in the appended claims. In a preferred embodiment, compositions for increasing the longevity of elderly cats are provided.

A first aspect of the invention provides the use of a nutritional composition comprising an oil blend a source of a prebiotic, and a source of at least one antioxidant in the manufacture of a medicament for increasing the longevity of an elderly pet, wherein the oil blend comprises oils high in omega 3 and omega-6 fatty acids, including linoleic acid.

Preferably, the antioxidant source is a vitamin source, optionally
(a) wherein the vitamin source is a carotenoid and optionally wherein the carotenoid is β-carotene; and/or
(b) wherein the vitamin source provides at least one of vitamin B1 and vitamin E.

Preferably,
(a) the nutritional composition includes a zinc source; and/or
(b) the nutritional composition is a nutritionally complete pet food; and/or
(c) the prebiotic is selected from the group consisting of inulin, fructooligosaccharides and plant materials which contain inulin and fructooligosaccharides; and/or
(d) the nutritional composition further comprises a source of calcium; and/or
(e) the oil blend is chosen from the group consisting of fish oil and plant oils; and/or
(f) the nutritional composition includes a protein source; and/or
(g) the nutritional composition includes chicory.

Preferably, the nutritional source comprises:
an oil blend at least one oil selected from the group consisting of fish oil and plant oil; and
vitamin E, beta carotene, and a protein source.

A second aspect of the invention provides the use of a nutritional composition comprising a calcium source, a zinc source, a protein source, a lipid source, a source of beta carotene, and a vitamin E source in the manufacture of a medicament for increasing the longevity of an elderly pet,
(a) wherein the nutritional composition further comprises a prebiotic, optionally wherein the prebiotic is selected from the group consisting of inulin, fructooligosaccharides and plant materials, which contain inulin and fructooligosaccharides; and/or
(b) wherein the nutritional composition comprising an oil blend selected from the group consisting of fish oil, soybean oil and sunflower oil.

A third aspect of the invention provides the use of a nutritional composition comprising whole chicory root, a source of gamma-linolenic acid, linoleic acid, protein, vitamin E, β-carotene, a fish oil a prebiotic selected from the group consisting of inulin, fructooligosaccharides and plant materials; and optionally a plant oil in the manufacture of a medicament for increasing the longevity of an elderly cat.

In embodiments of each of the above uses, additional components as set forth above may be used in nutritional compositions.

Typical nutritional compositions include a protein source, a lipid source, a calcium source, a zinc source, beta carotene, vitamin E, a source of linoleic acid and a prebiotic, optionally further comprising an oil blend and fish oil. The oil blend preferably includes at least one oil selected from the group consisting of sunflower oil, soybean oil and fish oil, and at least one antioxidant and a source of linoleic acid,
(a) wherein the linoleic acid is provided by the sunflower oil or soybean oil, and/or
(b) comprising a probiotic source.

A number of advantages are provided by the present invention including improving the longevity and health of elderly pets as well as providing improved pet food products.

Additional features and advantages of the present invention are described in, and will be apparent from, the following Detailed Description of the Invention and the figures.

### BRIEF DESCRIPTION OF THE FIGURES

Figures 1-3 illustrate, graphically, the results of the longevity trials of Example No. 1 at age 8, 11, and 14 respectively.
Figure 4 illustrates body weight over time (Age 10 - 15) from Example 1.
Figure 5 illustrates food consumption over time (Age 10 - 15) from Example 1.
Figure 6 illustrates longitudinal analysis of variance, Vitamin E (µg/ml) from Example No. 1.
Figure 7 illustrates longitudinal analysis of BUN from Example No. 1.

### DETAILED DESCRIPTION OF THE INVENTION

Embodiments of the invention are now described by way of example only. The invention is based upon the finding that the administration to an elderly cat of an effective amount of compositions of the present invention properties, improves the condition and/or longevity of the cat. As used herein, the term "elderly cat " or "senior cat" refers to a cat of age 7 years or more, although in certain cases, younger cats may exhibit characteristics and conditions generally associated with older animals. Although, at least certain of the compositions have been indicated to be effective in improving the longevity of senior cats.

The nutritional compositions are conveniently formulated into a pet food. The pet food may be any suitable pet food, for example a canned pet food, a semi-moist pet food, or an extruded and dried pet food. Further, the exact composition of the pet food is not critical.

The pet food contains a source of antioxidant(s). The antioxidant can be advantageously selected from the group of antioxidant vitamins and carotenoids. Suitable examples of such antioxidants and/or carotenoids include β-carotene, vitamin E and vitamin C. Preferably the pet food contains β-carotene and vitamin E.

If β-carotene is used, it may be present in an amount above about 4 mg/1000 kcal. For example, the nutritional composition may contain about 5 mg/1000 kcal of β-carotene. The vitamin E may be present in an amount of above about 75 IU/1000 kcal; for example, about 130 IU/1000 kcal.

The pet food contains a source of linoleic acid. Sources of linoleic acid include soybean oil and sunflower oil.

The pet food may also include a source of gamma linolenic acid. Alternatively, the oil blend includes at least one source of fish oil and/or sunflower oil and/or soybean oil.

The pet food may contain one or more protein sources. Any suitable protein source may be used. Suitable protein sources may be selected from any suitable animal, marine or plant protein source; for example, muscular or skeletal meat, meat and bone meal, poultry meal, fish meal, milk proteins, corn gluten, wheat gluten, soy flour, soy protein concentrates, soy protein isolates, egg proteins, whey, casein and gluten.

The amount of protein provided by the protein source may be selected as desired. For example, the pet food may contain from about 12% to about 70% by weight of protein on a dry matter basis.

The pet food may contain one or more carbohydrate sources. Any suitable carbohydrate source may be used. Preferably the carbohydrate source is provided in the form of grains, flours and starches. For example, the carbohydrate source may be rice, barley, sorghum, millet, oat, corn meal or wheat flour. Simple sugars such as sucrose, glucose and corn syrups may also be used.

The amount of carbohydrate provided by the carbohydrate source may be selected as desired. For example, the pet food may contain up to about 60% by weight of carbohydrate.

The pet food may contain one or more fat sources. Any suitable fat source may be used; fats from both animal and plant sources can be utilized. Examples of animal and marine fat sources that can be utilized are tallow, fish oil, poultry fat and pork fat. Plant oils such as corn oil, sunflower oil, soybean oil, safflower oil, canola oil and the like, may also be used. The fat source may include long chain fatty acids. Suitable long chain fatty acids include alpha-linolenic acid, gamma linolenic acid, linoleic acid, eicosapentanoic acid, and docosahexanoic acid. Fish oils are a suitable source of eicosapentanoic acids and docosahexanoic acid. Borage oil, blackcurrent seed oil and evening primrose oil are suitable sources of gamma linolenic acid. Safflower oils, sunflower oils, corn oils and soybean oils are suitable sources of linoleic acid. In one embodiment, the fat may be intrinsic to the raw petfood ingredients utilized in the manufacture of the petfood.

The amount of fat provided by the fat source may be selected as desired. For example, the pet food may contain about 5% to about 50% by weight of fat on a dry basis. The exact composition of the protein source, the carbohydrate source and the fat source will be selected based upon palatability, availability, cost and processing considerations.

The pet food may also include additional ingredients such as salts, spices, seasonings, flavoring agents, gums, and prebiotics. "Prebiotic" means a substance or compound which is fermented by the intestinal flora of the pet and hence promotes the growth or development of lactic acid bacteria, such as bifidobacteria and lactobacilli, in the gastro-intestinal tract of the pet at the expense of potentially pathogenic bacteria. The result of this fermentation is a release of fatty acids, in particular short-chain fatty acids in the colon. This has the effect of reducing the pH value in the colon. The prebiotics may be provided in any suitable form. For example, the prebiotic may be provided in the form of plant material, which contains the prebiotic. Suitable plant materials include asparagus, artichokes, onions, wheat, yucca or chicory, or residues of these plant materials. Alternatively, the prebiotic may be provided as an inulin extract or a hydrolyzed inulin extract. Extracts from whole chicory root are particularly suitable. Suitable prebiotics include oligosaccharides, such as inulin and its natural sources, fructo-oligosaccharides, galacto-oligosaccharides, xylo-oligosaccharides or oligo derivatives of starch. The prebiotics include products from inulin, hydrolyzed products, synthesized products, and resistant starch.

The pet food may be produced using any suitable process. Suitable processes for wet products include the following:
Process (i): To produce a thermally gelled emulsion, which sets upon cooling, a suitable meat material is comminuted to produce meat slurry. Suitable gelling agents, for example starches and gums such as kappa-carrageenan, locust bean gum, guar gum, and xanthan gum may be added to the meat slurry. Usually no more than about 1% by weight of gum is needed.

Water may also be added the meat batter to provide from about 70% to about 85% by weight of moisture. If sufficient moisture is present in the meat material, water need not be added.

The meat slurry is then heated to a temperature suitable to initiate thermal gelling of the mixture; for example a temperature of about 40°C to about 65°C in a mixer-cooker. Steam may be injected into the meat slurry if desired. The heated meat slurry may be emulsified if desired. The meat slurry is then maintained at a temperature of about 40°C to about 65°C until needed. After retorting and cooling to room temperature, the meat slurry forms a thermally gelled emulsion, which is substantially solid or at least holds its form.

Process (ii): To produce solid food pieces in gravy or gel, solid pieces of meat or other material, or both, may be mixed with a gravy. Solid pieces of other materials may also be used; such as rice grains, pasta or noodles, vegetable pieces, and the like.

The solid food pieces may be in the form of pieces of a thermally gelled matrix. The pieces of the thermally gelled matrix may be produced by any suitable procedure, for example the procedures described in any one of US patents 4,781,939, 5,132,137 and 5,567,466 and PCT application WO 97/02760.

The thermally gelled matrix may be formed in suitable equipment such as an emulsion mill or an extruder to form pieces or chunks. If an extruder is used, the emulsion may be forced through an orifice to provide the emulsion with a desired shape; for example of oval, square or rectangular cross-section. The shape and size of the piece may be variable. The extrudate may then be cooked in a suitable continuous cooking system; for example a tunnel oven using hot air, steam, mixtures of hot air and steam, or microwaves. The core temperature of the extrudate is raised such that the extrudate undergoes thermal gelling. For example, the core temperature may be raised to at least about 80°C; for example from about 85°C to about 95°C. The gelled extrudate may then be cut into pieces and the pieces cooled to provide pieces of a thermally gelled matrix. The pieces may be subjected to flaking (chipping or cutting off in flat thin pieces or layers) if desired. The cooling may be carried out by spraying water on the pieces. Alternatively, other cooling media may be used.

If gravy is used with the solid food pieces, it may be produced from water, one or more starch or gums, and suitable flavoring agents. The gravy preferably comprises about 20% to about 80% by weight of the mixture of solid pieces and gravy. Suitable gums are kappa-carrageenan, locust bean gum, guar gum, and xanthan gum.

If a gel is used with the solid food pieces, it may be produced from a suitable gelling agent, water and suitable flavoring agents. The gel preferably comprises about 20% to about 80% by weight of the mixture of solid pieces and gravy. Suitable gelling agents are proteins such as gelatin; gums such as alginates, kappa-carrageenan, locust bean gum, guar gum and xanthan gum, and the like. The gel or aspic may be prepared as is conventional.

Products that use a combination of the processes described above may also be used. For example, a thermally gelled emulsion may be prepared as described above. Then solid food pieces, which may be pieces of a thermally gelled matrix, meat pieces, vegetable pieces, combinations of these pieces, and the like, are combined with the thermally gelled emulsion. As a further alternative, combinations of thermally gelled emulsions and solid food pieces in gravy or gel may be used. Suitable combinations are described in WO 98/05218 and WO 98/05219; the disclosures of which are incorporated by reference.

The pet foods are then filled into cans or other containers, the containers sealed, and the products retorted in the normal manner, using suitable, commercially available equipment.

A suitable process for a dried pet food is cooking a feed mixture of the various ingredients, forming the cooked mixture into pellets, drying, and then coating the pellets with flavors. The cooking and forming steps are preferably carried out using an extruder, as is well known in the art. However, the pellets may be produced by other cooking procedures such as baking a preformed food body comprising the selected ingredients, preferably in nutritionally balanced proportions.

The amount of the pet food to be consumed by the pet to obtain a beneficial effect will depend upon the size of the pet, the type of pet and the age of the pet. By way of example and not limitation, an amount of the nutritional composition to provide a daily amount of at least about 5mg/1000kcal β-carotene and at least about 100 IU/1000kcal of vitamin E, would usually be adequate.

Numerous modifications may be made to the embodiments described above without departing from the scope of the invention. By way of example and not limitation, examples of the present invention are now given.

A preliminary study, as described in WO 01/17366, was conducted to determine the effect of antioxidants and other nutrients on antioxidant status, bone health, and signs of aging in elderly cats. After 6 months, cats consuming the diet supplemented with antioxidants showed significantly higher indicators of antioxidant status than cats consuming the unsupplemented diets, and all cats fed the supplemented diet survived to the completion of the trial (9 months), while some deaths were noted on the other treatment groups. The trial showed that adding antioxidants and perhaps other supplements to the diet may affect a cat's longevity.

### Example No. 1

A longer-term study was initiated with a large population of senior cats, with the objective to evaluate whether antioxidants and other nutritional supplements increase longevity and enhance quality of life. Statistically significant differences in survival, e.g., longevity, require a larger number of animals be assigned to each test group. Therefore, a total of 90 cats were utilized in the current trial.

### Protocol

A total of 30 cats per diet were initiated on trial, spread equally between three age categories: 7 to 9 years, 10 to 12 years, and 13+ years. Cats were assigned to one of three diets:
**Diet 1**: Basal diet: Standard wet fish-based cat food (Control Diet)
**Diet 2:** Basal diet + antioxidants (β-carotene, 5 mg/1000 kcal; vitamin E, 100 ICJ/1000 kcal)
**Diet 3:** Basal diet + antioxidants (β-carotene, 5 mg/1000 kcal; vitamin E, 100 IU/1000 kcal) + oil blend (a source of omega 6 and omega 3 fatty acids) + at least 0.5% whole chicory root. The diets were fed for the remaining life of each cat assigned to the trial.

The following initial measurements were taken on all cats: complete blood count, serum chemistries, plasma fatty acid profile (control and Diet 3 only), antioxidant status (vitamin E, beta carotene), and fecal microflora (control and Diet 3 only). Cats were also given complete physical examinations and assigned a body condition score. Food consumption was measured daily during the study, and body weights were assessed weekly. Measurements taken at study initiation were repeated every three to six months.

Cats were split into 3 study groups (blocks) and stagger-started on trial over a 6-month period. Cats were blocked for assignment to diets on the basis of gender, age, body condition score, and initial health status.

### Results

Data analysis showed no statistically significant between-group differences in any of the baseline measures, indicating that randomization was effective in producing balance at baseline in the three study groups.

*Longevity:* Table 1 shows the deaths from each diet and phase after 4.3 years into the study. A total of 59 cats had died at this point in the study.

**Table 1. Deaths by Diet and Phase (Age at Baseline).**

| | **AGE AT BASELINE** | | | |
|---|---|---|---|---|
| ***DIET*** | ***13-17 YEARS*** | ***10-12 YEARS*** | ***7-9 YEARS*** | ***TOTAL DEATHS*** |
| 1 | 9 | 6 | 3 | 18 |
| 2 | 10 | 9 | 4 | 23 |
| 3 | 8 | 8 | 2 | 18 |
| TOTAL DEATHS | 27 | 23 | 9 | 59 |

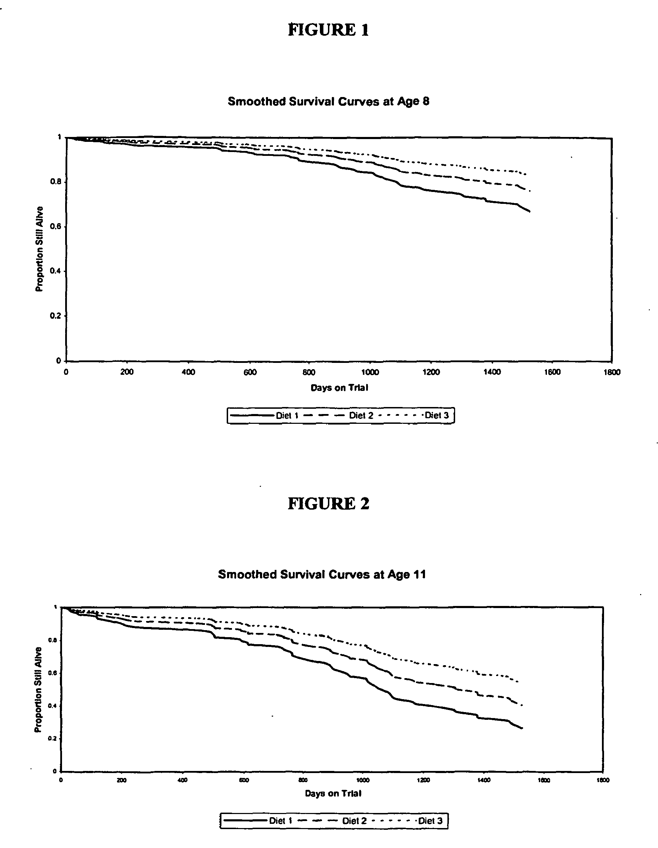

To evaluate potential differences in survival between diets, a Cox Proportional Survival Hazard Model was performed to compare survival curves, using the number of days a cat was on trial until it died. Although the number of deaths per group as shown in Table 1 do not seem different, survival analysis showed significant dietary differences. Table 2 gives the results of the Cox Proportional Hazard model using initial age of the cat as a covariate, since some cats started the trial at different ages. Results of the model show that Diet 3 is significantly different than Diet 1, whether the starting age for the cat was 8, 11, or 14 years. In this model, the hazard ratio of Diets 1 vs 3 was 0.40, meaning that the hazard of dying for the cats on Diet 3 is only 40% of the hazard of dying for the cats on Diet 1. There was not a significant difference between Diets 1 and 2, or between Diets 2 and 3. Figures 1, 2, and 3 show the smoothed survival curves from the Proportional Hazard model using initial ages of 8, 11 and 14 years. For all starting ages, the analyses show that the proportion of cats surviving is significantly higher on Diet 3 than Diet 1. Several analyses were performed on the survival data. These analyses were the Kaplan Meier, Proportional Hazard, and Accelerated Failure Time. The purpose of the analyses was to determine if there were any problems with the model comparing diets using age of the cats as a covariate. The results of the analyses indicate that the statistical model was valid, and that the cats are living longer on Diet 3.

**Table 2. Survival analysis**

| ***Variable*** | ***DF*** | ***P-value*** | ***Hazard Ratio*** | ***95% CI for Hazard Ratio*** |
|---|---|---|---|---|
| Diet 1 v Diet 2 | 1 | 0.6677 | 0.868 | 0.456-1.654 |
| Age | 1 | <.0001 | 1.473 | 1.245-1.743 |
| | | | | |
| Diet 1 v Diet 3 | 1 | 0.0132 | 0.400 | 0.194-0.825 |
| Age | 1 | <.0001 | 1.572 | 1.322-1.869 |
| | | | | |
| Diet 2 v Diet 3 | 1 | 0.1317 | 0.619 | 0.331-1.155 |
| Age | 1 | <.0001 | 1.413 | 1.212-1.649 |

Table 3 gives the results of censored regressions comparing the diets for age at death. These results show that the cats on Diet 3 are living significantly longer than the cats on Diets 1 and 2. Examining the predicted age of death for different initial ages, cats on Diet 3 are living about 1 year longer on average than cats in the other groups (Table 4).

**Table 3. Comparison of Diets for Age at Death.**

| ***Effect*** | ***DF*** | ***Wald Chi Square*** | ***P-value*** |
|---|---|---|---|
| All Diets | 2 | 7.1683 | 0.0279 |
| Diet 1 v Diet 2 | 1 | 0.0112 | 0.9158 |
| Diet 1 v Diet 3 | 1 | 6.8414 | 0.0089 |
| Diet 2 v Diet 3 | 1 | 5.4219 | 0.0199 |
| Initial Age | 1 | 54.0185 | <0.0001 |

**Table 4. Predicted Mean Age at Death at Different Initial Ages (Years)**

| Initial Age = 8 | | |
|---|---|---|
| Diet | Predicted Mean | Standard Error |
| 1 | 12.5588 | 0.41977 |
| 2 | 12.6211 | 0.43212 |
| 3 | 13.5621 | 0.46584 |

| Initial Age = 11 | | |
|---|---|---|
| Diet | Predicted Mean | Standard Error |
| 1 | 14.1500 | 0.30667 |
| 2 | 14.2122 | 0.30172 |
| 3 | 15.1533 | 0.33247 |

| Initial Age = 14 | | |
|---|---|---|
| Diet | Predicted Mean | Standard Error |
| 1 | 15.7412 | 0.32501 |
| 2 | 15.8034 | 0.29847 |
| 3 | 16.7445 | 0.31273 |

*Morbidity*/*Pathology:* Morbidity data were recorded and tabulated to determine time from initial clinical sign of illness to time of death. There was an indication (p < 0.10) that the cats on Diet 3 survived longer after initial signs of disease were recorded (Table 5).

**Table 5. Comparison of Diet for Time to Death (in Years)**

| ***Diet*** | ***Mean*** | ***SD*** | ***Tukey Adjusted p-vαlues*** | |
|---|---|---|---|---|
| 1 | 0.6456 | 0.6004 | Diet 1 v Diet 2 | 0.6877 |
| 2 | 0.8526 | 0.9355 | Diet 1 v Diet 3 | 0.0877 |
| 3 | 1.2172 | 0.7677 | Diet 2 v Diet 3 | 0.3192 |

Pathology results of deceased cats were recorded and tabulated for six general pathologies (renal, pancreas, cancer of any type, thyroid, gastrointestinal, and liver). Half of the Diet 1 cats (9 out of 18) had thyroid pathology recorded at necropsy, compared with only about a fourth of cats from Diet 3 (4 out of 17 cats), even though the average age at death was 1.5 years greater in Diet 3 than for Diet 1. Likewise, half of the Diet 1 cats (9 out of 18) had some form of gastrointestinal pathology compared with only about a fourth of Diet 3 cats (4 out of 17 cats). For Diet 2, the number of cats with these pathologies was intermediate between Diet 1 and Diet 3. For the other pathologies, similar numbers of cats were affected in each of the three diets.

In the Competing Risk analysis cats were grouped according to whether they died with thyroid and/or GI pathologies (Thyroid/GI Deaths). Table 6 gives the results of the Cox's Proportional Hazard model for each group. This analysis resulted in a significant difference (p=0.0009) between the models for the two groups of cats. Examination of the models showed no difference in survival between Diets 1 and 3 for those cats without thyroid or GI pathology. These results indicate that cats with thyroid or gastrointestinal pathology survived longer when consuming Diet 3 than cats consuming Diet 1. Comparisons involving Diet 2 did not show any statistically significant differences.

**Table 6. Comparison of Incidence of Pathologies (Diets 1 and 3).**

| Non-Thyroid/GI Deaths | | | | |
|---|---|---|---|---|
| Variable | DF | P-value | Hazard Ratio | 95% CI for Hazard Ratio |
| Diet 1 v Diet 3 | 1 | 0.6294 | 0.778 | 0.281 - 2.157 |
| Initial Age | 1 | 0.0049 | 1.390 | 1.105 - 1.749 |

| Thyroid/GI Deaths | | | | |
|---|---|---|---|---|
| Variable | DF | P-value | Hazard Ratio | 95% CI for Hazard Ratio |
| Diet 1 v Diet 3 | 1 | 0.0028 | 0.199 | 0.064-0.566 |
| Initial Age | 1 | <0.0001 | 1.822 | 1.390-2.389 |
| p-value for comparing Thyroid/GI deaths to Non-Thyroid/GI deaths = 0.0009 | | | | |

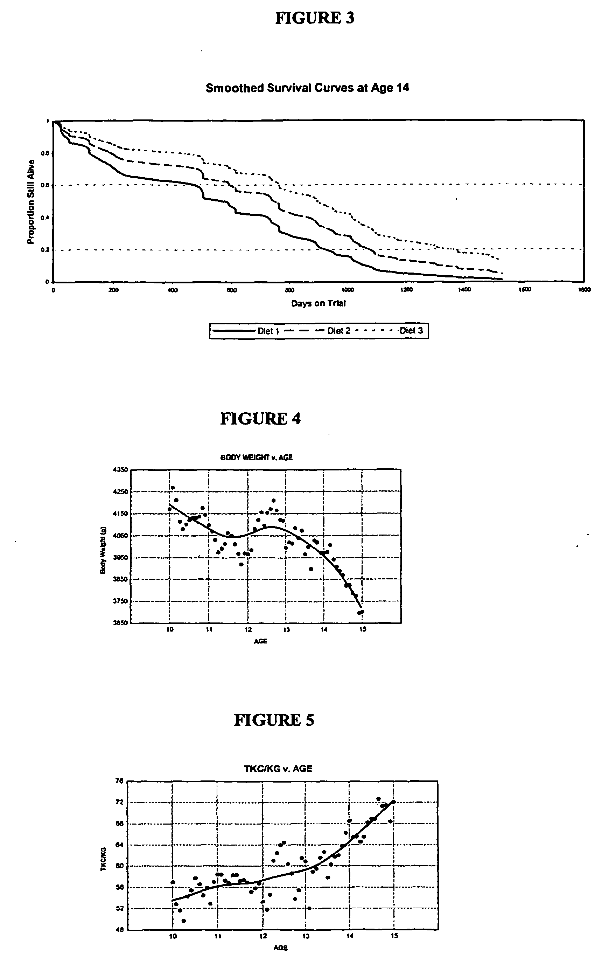

*Weight Maintenance and Food Consumption:* All three groups lost weight over time, on average, which is expected with aging cats. Average weight losses and food consumption were not significantly different between the three dietary treatment groups. Food consumption was steady on a grams-per-day basis, but calories per kilogram body weight increased over time. Food consumption increased from 10 to 15 years of age for a cat (irrespective of diet) while the body weight decreased. Figure 4 illustrates body weight over time (Age 10-15) and Figure 5 illustrates food consumption over time (Age 10-15). In this trial, no differences in food consumption were seen between the three diets. *Microflora:* Initial, 1-month and 3-month fecal microflora results showed a significant increase in *Bifidobacteria* for the cats on Diet 3 as well as a significant decrease in *Clostridia* bacteria; whereas there were no statistically significant changes in fecal microflora for the cats on Diet 1 (Control). A 77% of the cats on the chicory containing diet responded with either an increase in "desirable" bacteria (*Bifidobacteria, Lactobacillus*) and/or a decrease in the number of "undesirable" bacteria (*Clostridia*).
*Blood.* Cats fed Diet 3 had significantly increased levels of serum vitamin E, serum β-carotene, and plasma linoleic acid and plasma alpha-linolenic acid compared to cats eating the control diet. Table 7 shows the means and p-values from the ANOVA for beta-carotene, linoleic acid, and alpha-linolenic acid after 6 months on trial.

**Table 7. ANOVA, Linoleic Acid, α-Linolenic Acid, and β-Carotene**

| ***Dependent Variable*** | ***Diet*** | ***Mean SE*** | ***Initial*** | ***3 Months*** | ***6 Months*** | ***P-values*** | |
|---|---|---|---|---|---|---|---|
| Plasma Linoleic Acid (g/100g fat) | 1 | Mean | 21.38 | 19.89 | 19.17 | Diet | 0.00000 |
| | | SE | 0.5 | 0.5 | 0.52 | Time | 0.00000 |
| | 3 | Mean | 20.04 | 26.89 | 26.74 | Diet*Time | 0.00000 |
| | | SE | 0.41 | 0.41 | 0.43 | | |
| | | SE | 0.04 | 0.04 | 0.04 | | |
| Plasma α-Linolenic Acid (g/100g fat) | 1 | Mean | 0.32 | 0.30 | 0.23 | Diet | 0.01505 |
| | | SE | 0.05 | 0.05 | 0.05 | Time | 0.04275 |
| | 2 | Mean | 0.30 | 0.49 | 0.37 | Diet*Time | 0.03234 |
| | | SE | 0.04 | 0.04 | 0.04 | | |

| ***Dependent Variable*** | ***Diet*** | ***Mean SE*** | ***Initial*** | ***3 Months*** | **6 *Months*** | Diet*Time Comparison | P-value |
|---|---|---|---|---|---|---|---|
| Serum Beta Carotene (µg/ml) | 1 | Mean | 0.00 | 0.07 | 0.04 | Diet 1 v 2 3 months | 0.04128 |
| | | SE | 0.02 | 0.02 | 0.02 | Diet 1 v 2 6 months | 0.00211 |
| | 2 | Mean | 0.03 | 0.13 | 0.13 | Diet 1 v 3 3 months | 0.00731 |
| | | SE | 0.02 | 0.02 | 0.02 | Diet 1 v 3 6 months | 0.00001 |
| | 3 | Mean | 0.02 | 0.15 | 0.17 | Diet 2 v 3 3 months | 0.00731 |
| | | SE | 0.02 | 0.02 | 0.02 | Diet 2 v 3 6 months | 0.00001 |

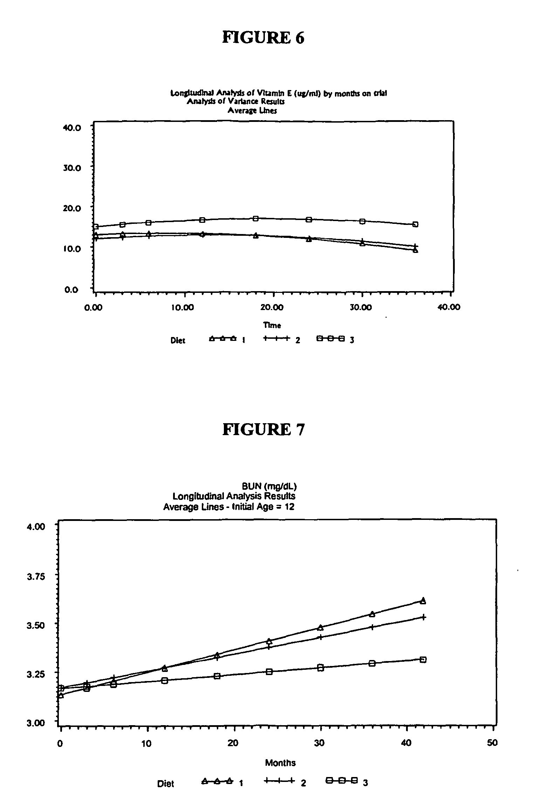

Serum Vitamin E levels were evaluated at 0, 3, 6, 9, 24, 30, 36, 42 and 48 months on trial and a longitudinal analysis of variance performed relating vitamin E to time by diet. Figure 6 shows a plot of the predicted means for each diet for an initial age of 12 years. There was a significant diet-time interaction (p < 0.05), and Diet 3 cats had significantly higher serum levels of vitamin E than either Diets 1 or 2 (p < 0.05). The curves show that the difference between Diet 3 and the other diets is increasing with time, and comparison of the slopes showed that Diets 1 and 3 were significantly different (p < 0.05).

The differences between Diet 3 and Diet 1 (control) are the antioxidants, source of prebiotic and added oils, resulting in a different blood fatty acid profile; specifically, there was a significantly higher level of linoleic acid and α-linolenic acid in the plasma of cats eating Diet 3. This fatty acid profile was achieved due to a level of approximately 20% linoleic acid and 2% α-linolenic acid (of total fat) in Diet 3 compared to a level of 9.4% linoleic acid and 1% α-linolenic acid (of total fat) in the control diet. Although both test diets contained the same dietary levels of antioxidants, only the cats eating Diet 3 with the added oil blend and chicory showed significantly increased serum vitamin E, a marker of improved antioxidant status. Figure 6 illustrates longitudinal analysis of variance, vitamin E (µg/ml).
Longitudinal analysis of variance relating serum urea nitrogen (BUN) to time showed a significant diet-time interaction at the 0.10 level, with Diet 3 cats having lower levels than either Diets 1 or 2. The slopes showed that BUN significantly increased with time for Diets 1 and 2 (p < 0.01) while for Diet 3 there was no significant change over time (p > 0.10). The difference in slopes for Diet 1 vs Diet 3 was significantly different (p < 0.05). Figure 7 illustrates longitudinal analysis of BUN.

### Conclusions

Figures 1-3 illustrate graphically the longevity of the cats on the different diets. Cats on Diet 3 are living longer than cats on Diet 1, when initial age is used as a covariate. The cats on Diet 3 have a 40% lower risk of dying and live at least one year longer than cats on Diet 1.

In summary, senior cats fed a diet containing supplemental antioxidants vitamin E and β-carotene, whole chicory root, and a blend of n-3 and n-6 fatty acids lived significantly longer than cats eating a standard nutritionally complete feline diet. Of the cats that died, positive effects on morbidity and pathology suggests that the nutrient cocktail may provide some protection against certain disease states that may contribute to their increased longevity. Data from this study indicates positive effects of Diet 3 on longevity, antioxidant status, intestinal health, renal disease, and thyroid function. The study indicated that the risk of dying is lower for cats fed Diet 3 compared to the control diet (Diet 1). The cats fed Diet 3 lived at least one year longer than cats fed the control diet.

## Claims

1. Use of a composition comprising an oil blend, a source of anti-oxidants and a source of prebiotic in the manufacture of a medicament for increasing the longevity of an elderly pet, wherein the oil blend comprises oils high in omega 3 and omega 6 fatty acid, including linoleic acid.

2. Use according to claim 1, wherein the source of antioxidants is selected from the group consisting of vitamins and carotenoids.

3. Use according to claim 2, wherein the antioxidant is selected from one or more of the group consisting of Vitamin C, Vitamin E and β-carotene.

4. Use according to claim 2 or claim 3, wherein the antioxidant source comprises Vitamin E and β carotene.

5. Use according to claim 4, wherein the composition comprises 4 mg/1000 KCal to 5 mg/1000 KCal β-carotene and 75 to 130 IU/1000 KCal Vitamin E.

6. Use according to any one of claims 1 to 5, wherein the prebiotic source is selected from one or more of the group comprising inulin, oligo-saccharides, fructooligosaccharides, soluble fibres, pectin, fermentable fibres and plant materials containing inulin and/or oligosaccharides.

7. Use according to any one of claims 1 to 6, wherein the composition is a nutritionally complete pet food.

8. Use according to any one of claims 1 to 7, wherein the composition comprises at least 0.5% by weight whole chicory root, an oil blend including a source of omega-6 and omega-3 fatty acids, 100 IU/1000 KCal Vitamin E, 5 mg/1000 KCal β-carotene, wherein the all blend comprises 20% by weight linoleic acid and 2% by weight linolenic acid.

## Patentansprüche

1. Verwendung einer Rezeptur, die eine Ölmischung, eine Quelle an Antioxidationsmitteln und eine Quelle an Präbiotika aufweist, bei der Herstellung eines Medikamentes zur Erhöhung der Langlebigkeit eines älteren Haustieres, wobei die Ölmischung Öle, die reich an Omega-3 und Omega-6 Fettsäuren sind, einschließlich Linolsäure aufweist.

2. Verwendung nach Anspruch 1, wobei die Quelle an Antioxidationsmitteln aus einer Gruppe ausgewählt ist, die Vitamine und Karotinoide aufweist.

3. Verwendung nach Anspruch 2, wobei das Antioxidationsmittel aus einer oder mehreren Gruppen ausgewählt ist, die Vitamin C, Vitamin E und β-Karotin aufweisen.

4. Verwendung nach Anspruch 2 oder 3, wobei die Quelle an Antioxidationsmitteln Vitamin E und β-Karotin aufweist.

5. Verwendung nach Anspruch 4, wobei die Rezeptur 4 mg/1000 Kcal bis 5 mg/1000 Kcal β-Karotin und 75 bi 130 IU/1000 Kcal Vitamin E aufweist.

6. Verwendung nach einem der Ansprüche 1 bis 5, wobei die Quelle an Präbiotika aus einer oder mehreren der Gruppen ausgewählt ist, die Inulin, Oligosaccharide, Fructooligosaccharide, lösliche Ballaststoffe, Pektin, gärfähige Ballaststoffe und pflanzlichen Stoffe, die Inulin oder Oligosaccharide enthalten, aufweisen.

7. Verwendung nach einem der Ansprüche 1 bis 6, wobei die Rezeptur ein vollwertiges Tierfutter ist.

8. Verwendung nach einem der Ansprüche 1 bis 7, wobei die Rezeptur mindestens 0,5 Gewichtsprozent vollständige Schikoreewurzeln, eine Ölmischung mit einer Quelle an Omega-3 und Omega-6 Fettsäuren, 100 IU/1000 Kcal Vitamin E, 5 mg/1000 Kcal β-Karotin aufweist, wobei die Ölmischung 20 Gewichtsprozent Linolsäure und 2 Gewichtsprozent Linolensäure aufweist.

## Revendications

1. Utilisation d'une composition chimique comprenant un mélange huileux, une source d'anti-oxydants et une source de prébiotiques pour la fabrication d'un médicament permettant d'augmenter la longévité d'un animal domestique vieillissant, dans lequel le mélange huileux comprend des huiles chargées en acides gras oméga 3 et oméga 6, y compris l'acide linoléique.

2. Utilisation selon la revendication 1, dans laquelle la source d'anti-oxydants est sélectionnée dans le groupe comprenant vitamines et caroténoïdes.

3. Utilisation selon la réclamation 2, dans laquelle l'anti-oxydant est sélectionné dans un ou plusieurs éléments du groupe comprenant Vitamine C, Vitamine E et β-carotène.

4. Utilisation selon la revendication 2 ou 3, dans laquelle la source d'anti-oxydant comprend de la Vitamine E et du β-carotène.

5. Utilisation selon la revendication 4, dans laquelle la composition comprend 4 mg/1000 Kcal à 5 mg/1000 Kcal de β-carotène et 75 à 130 IU/1000 Kcal de Vitamine E.

6. Utilisation selon une quelconque des revendications 1 à 5, dans laquelle la source prébiotique est sélectionnée parmi un ou plusieurs éléments du groupe comprenant l'inuline, les oligo-saccharides, les fructooligosaccharides, des fibres solubles, la pectine, des fibres fermentescibles, et des éléments végétaux contenant de l'inuline et/ou des oligosaccharides.

7. Utilisation selon une quelconque des revendications 1 à 6, dans laquelle la composition est une nourriture pour animaux domestiques nutritionnellement complète.

8. Utilisation selon une quelconque des revendications 1 à 7, dans laquelle la composition comprend au moins 0,5% en poids de racine de chicorée, un mélange huileux comprenant une source d'acides gras oméga 3 et 6, dans laquelle le mélange huileux comprend 20% en poids d'acide linoléique et 2% d'acide linolénique.
